# EUROPÄISCHE PATENTSCHRIFT

(11) **EP 0 500 005 B1**
(45) Veröffentlichungstag und Bekanntmachung des Hinweises auf die Patenterteilung: **07.02.1996**
(21) Anmeldenummer: 92102593.8
(22) Anmeldetag: 17.02.1992
(51) Int. Cl.: C07C 2/86, C07C 17/26, C07C 13/465, C07C 25/22, C07D 307/36, C07D 307/42, C07D 213/16, C07D 233/08

(54) **Verfahren zur Herstellung substituierte Indene**
Process for the preparation of substituted indenes
Procédé de préparation des indènes substitués

(30) Priorität: 18.02.1991 DE 4104931
(43) Veröffentlichungstag der Anmeldung: 26.08.1992
(73) Patentinhaber: HOECHST AKTIENGESELLSCHAFT, D-65926 Frankfurt am Main (DE)
(72) Erfinder: Reuschling, Dieter Bernd, Dr., W-6308 Butzbach (DE); Rohrmann, Jürgen, Dr., W-6233 Kelkheim (Taunus) (DE); Erker, Gerhard, Prof. Dr., D-4400 Münster (DE); Nolte, Reiner, Dr., W-6700 Ludwigshafen (DE); Aulbach, Michael, Dr., W-6233 Kelkheim (Taunus) (DE); Weiss, Astrid, W-4400 Münster (DE)

(56) Entgegenhaltungen:
- US-A- 3 210 433
- US-A- 4 677 238
- BULLETIN DE LA SOCIETE CHIMIQUE DE FRANCE. Bd. 6, 1969, PARIS FR Seiten 1981 -1989; E. MARECHAL ET AL.: 'Homopolymérisation cationique des diméthyl-4,7,diméthyl-4,6 et diméthyl-5,6 indènes'

## Beschreibung

Die vorliegende Erfindung betrifft ein Verfahren zur einstufigen Herstellung von substituierten Indenen.

Verbindungen dieses Typs eignen sich vorteilhaft als Ligandsysteme zur Herstellung von chiralen, stereorigiden Metallocen-Komplexen. Insbesondere die entsprechenden Zirkon-Derivate besitzen als hochaktive Katalysatoren bei der Olefinpolymerisation Bedeutung (vgl. EP-A 129 368). Durch Variation des Ligandsystems, z.B. durch Substitution, können die Katalysatoreigenschaften gezielt beeinflußt werden. Hierdurch ist es möglich, die Polymerausbeute, die Molmasse, die Taktizität oder den Schmelzpunkt der Polymeren im gewünschten Maß zu verändern (New J. Chem. 14 (1990) 499; J. Am. Chem. Soc. 112 (1990) 2030; Angew. Chem. 102 (1990) 339; Chem. Lett. (1989) 1853; EP-A 316 155; EP-A 351 392).

Weiterhin können Indene auch als Monomere bei der Homopolymerisation oder Copolymerisation mit anderen Olefinen, wie z.B. Styrol, eingesetzt werden (vgl. Macromol. 22 (1989) 3824; Bull. Soc. Chim. Fr. 6 (1969) 2039).

Die wenigen in der Literatur beschriebenen sechsringsubstituierten Indene sind jedoch in der Regel nur über vielstufige Synthesen in geringen Ausbeuten zugänglich. Meistens werden diese Indenderivate durch Ankondensieren des Fünfrings an einen entsprechend substituierten Aromaten in ca. 5 Synthesestufen hergestellt. Bestimmte Substitutionsmuster sind auf diesem Wege nicht zugänglich (Bull. Soc. Chim. Fr. (1969), 6, 1981-89,
Acta Chem. Scand. B 30 (1976) 527-32,
Austr. J. Chem. 29 (1970) 2572,
Chem. Lett. (1981) 729-730,
Ber. 97 (12), (1964) 3461-8).

Es bestand somit die Aufgabe, ein Verfahren zur Herstellung der obengenannten Indene zu finden, das die aus dem Stand der Technik bekannten Nachteile vermeidet.

Diese Aufgabe wird erfindungsgemäß dadurch gelöst, daß in einem technisch einfach zu handhabenden Einstufenprozeß Cyclopentadiene mit Diketonen bzw. Ketoaldehyden zu den gewünschten substituierten Indenen umgesetzt werden. Gleichzeitig ermöglicht das erfindungsgemäße Verfahren die Herstellung von neuen Verbindungen des genannten Strukturtyps.

Die vorliegende Erfindung betrifft daher ein Verfahren zur Herstellung einer Verbindung der Formel I oder deren Isomer der Formel Ia 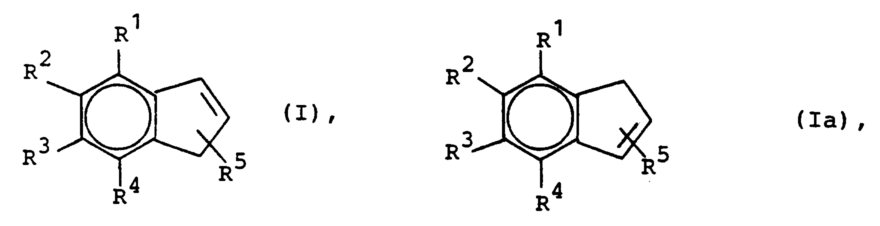
worin
- R¹: (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃ oder einen heteroaromatischen Rest mit 5 oder 6 Ringgliedern, der ein oder mehrere Heteroatome enthaften kann, bedeutet,
- R², R³ und R⁴: gleich oder verschieden sind und neben Wasserstoff die für R¹ genannten Bedeutungen besitzen,
- R⁵: Wasserstoff, (C₁-C₁₀)Alkyl, (C₁-C₁₀)Fluoralkyl oder (C₂-C₁₀)Alkenyl bedeutet und
- R⁶: für (C₁-C₁₀)Alkyl steht,
dadurch gekennzeichnet, daß man eine Verbindung der Formel II 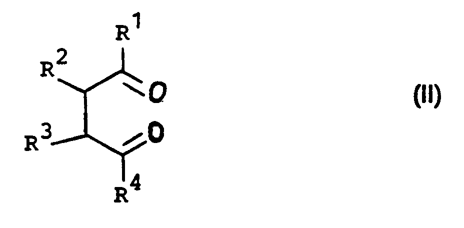
mit einer Verbindung der Formel III 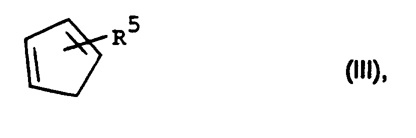
wobei die Substituenten R¹-R⁵ die genannten Bedeutungen besitzen, in Gegenwart einer Base umsetzt.

Alkyl steht für geradkettiges oder verzweigtes Alkyl. Halogen bedeutet Fluor, Chlor, Brom oder Jod, insbesondere Fluor oder Chlor. Beispiele für heteroaromatische Reste sind Thiophenyl, Furyl oder Pyridyl.

In den Formeln I bzw. Ia gilt bevorzugt, daß
- R¹: (C₁-C₁₀)Alkyl, Phenyl, (C₁-C₄)Alkoxy, (C₂-C₆)Alkenyl, (C₇-C₁₀)Arylalkyl, (C₇-C₁₀)Alkylaryl, Phenoxy, (C₁-C₆)Fluoralkyl, Halogenphenyl, (C₂-C₆)Alkinyl, einen Rest -SiR⁶₃ oder einen heteroaromatischen Rest mit 5 oder 6 Ringgliedern, der ein oder mehrere Sauerstoff-, Schwefel- und/oder Stickstoffatome enthalten kann, bedeutet,
- R², R³ und R⁴: gleich oder verschieden sind und neben Wasserstoff die für R¹ genannten Bedeutungen besitzen,
- R⁵: Wasserstoff, (C₁-C₆)Alkyl, (C₁-C₆)Fluoralkyl oder (C₂-C₆)Alkenyl bedeutet und
- R⁶: für (C₁-C₆)Alkyl steht.

Insbesondere gilt, daß
- R¹: (C₁-C₁₀)Alkyl, Phenyl, C₂-Alkenyl, (C₇-C₁₀)Arylalkyl, (C₇-C₁₀)Alkylaryl, (C₁-C₆)Fluoralkyl, Halogenphenyl, einen Rest -SiR⁶₃ oder einen heteroaromatischen Rest mit 5 oder 6 Ringgliedern, der ein Sauerstoff-, Schwefel- oder Stickstoffatom enthält, bedeutet,
- R², R³ und R⁴: gleich oder verschieden sind und neben Wasserstoff die für R¹ genannten Bedeutungen besitzen,
- R⁵: Wasserstoff, (C₁-C₃)Alkyl, (C₁-C₃)Fluoralkyl oder (C₂-C₃)Alkenyl bedeutet und
- R⁶: für Methyl oder Ethyl steht.

Die Herstellung der Ausgangsverbindungen II ist literaturbekannt (Diketone: Chem. Ber. 114, 1226 (1981), ibid. 109, 3426 (1976), ibid. 107, 2453 (1974); Ketoaldehyde: Synthesis 1985, 1058).

Die Cyclopentadiene III sind im Handel erhältlich oder können nach bekannten Methoden, z.B. durch Alkylierung am Cyclopentadienanion, hergestellt werden.

Die substituierten Indene fallen als Doppelbindungsisomere an (I/Ia, vgl. Tab. 1). Wird ein substituiertes Cyclopentadien III (R⁵ ≠ H) eingesetzt, dann können zusätzlich Konstitutionsisomere entstehen, die sich säulenchromatographisch trennen lassen. Die Doppelbindungsisomere (Gemisch) können direkt für die Synthese der entsprechenden Metallocenkomplexe eingesetzt weden; Konstitutionsisomere sind vor der Folgereaktion zu trennen.

Die Umsetzung wird in einem inerten Lösemittel durchgeführt. Bevorzugt werden Alkohole wie Methanol, Ethanol oder t-Butanol verwendet, insbesondere Methanol.

Als Basen für das erfindungsgemäße Verfahren lassen sich eine Vielzahl von Verbindungen verwenden. Als Beispiele seien genannt: Alkali- und Erdalkalihydroxide, Alkali- und Erdalkalialkoholate, wie Natriummethanolat, Natriumethanolat, Kaliumtertiärbutylat, Amide wie Lithiumdiisopropylamid oder Amine. Bevorzugt unter diesen Verbindungen sind Natriummethanolat, Kaliumtertiärbutylat und Kaliumhydroxid.

Die Molverhältnisse der Ausgangsverbindungen einschließlich der zu verwendenden Base können innerhalb weiter Grenzen schwanken. Bevorzugt ist das Molverhältnis von Verbindung II : III: Base = 1 : 1-1,5 : 2-3; insbesondere 1 : 1,1 : 2,5.

Die Reaktionstemperatur beträgt bevorzugt -40 °C bis 100 °C, insbesondere 0 °C - 25 °C.

Die Reaktionszeiten schwanken in der Regel zwischen 10 min. und 100 h, vorzugsweise zwischen 1 h und 30 h.

Bevorzugt wird eine Mischung der Verbindungen II und III - evtl. in einem Lösemittel - zu der vorgelegten Lösung, die aus Base und Lösemittel besteht, gegeben.

Das erfindungsgemäße Verfahren zeichnet sich besonders dadurch aus, daß der aromatische Sechsring des substituierten Indengerüsts in einem Reaktionsschritt aufgebaut wird und dadurch eine große Anzahl unterschiedlicher Substitutionsmuster sehr einfach realisiert werden kann, die auf konventionellem Weg nicht oder nur schwer zugänglich ist.

Die nachfolgenden Beispiele dienen zur näheren Erläuterung der Erfindung.

Allgemeine Angaben: Herstellung und Handhabung organometallischer Verbindungen erfolgten unter Ausschluß von Luft und Feuchtigkeit unter Argon-Schutz (Schlenk-Technik). Alle benötigten Lösungsmittel wurden vor Gebrauch durch mehrstündiges Sieden über einem geeigneten Trockenmittel und anschließende Destillation unter Argon absolutiert.
Die Herstellung der als Ausgangsverbindungen eingesetzten Diketone und Ketoaldehyde erfolgte nach literaturbekannten Methoden. Cyclopentadien und Methylcyclopentadien wurden durch Cracken der Dimeren gewonnen und bei -35 °C gelagert.

### Beispiel 1: 4,7-Dimethylinden (1)

34,4 g (1,50 mol) Natrium wurden in 300 ml absolutem Methanol gelöst. Bei 0 °C wurde ein Gemisch aus 53,8 ml (43,6 g, 0,66 mol) Cyclopentadien und 67,3g (0,59 mol) 2,5-Hexandion langsam zugetropft. Nach beendeter Zugabe wurde noch eine Stunde bei 0 °C, anschließend noch zwei Stunden bei Raumtemperatur gerührt. Die Reaktionsmischung wurde mit 200 ml Wasser und 300 ml Diethylether versetzt. Die organische Phase wurde im Scheidetrichter abgetrennt und die wässrige Phase zweimal mit jeweils 50 ml Diethylether gewaschen. Die vereinigten organischen Phasen wurden zweimal mit jeweils 50 ml Wasser gewaschen und anschließend über Natriumsulfat getrocknet. Das Lösungsmittel wurde im Vakuum entfernt und der ölige Rückstand fraktionierend destilliert (48-52°C, 0,1 Torr). Ausb. 48,8 g (56 %). NMR-Daten siehe Tabelle 1.

### Beispiel 2: 4,7-Dimethylinden (1)

23 g (1,0 mol) Natrium wurden in 250 ml absolutem Methanol gelöst. Bei 0 °C wurde eine Mischung aus 45,6 g (0,40 mol) 2,5-Hexandion und 39,7 g (0,60 mol) Cyclopentadien innerhalb 1-2 h zugetroft. Nach 1 h Rühren bei Raumtemperatur wurde die dunkelbraune Lösung mit 50 ml H₂O versetzt. Die organische Phase wurde mit ca. 1 l Diethylether verdünnt. Die wässrige Phase wurde abgetrennt. Die organische Phase wurde über Natriumsulfat getrocknet und eingedampft. Das verbleibende Öl wurde an Kieselgel 60 chromatographiert. Mit Hexan/Methylenchlorid (10:1) ließen sich 44,2 g (76 %) des Indens 1 eluieren, das nach Abziehen des Laufmittels im Ölpumpenvakuum als farbloses bis hellgelbes Öl anfällt. NMR-Daten siehe Tabelle 1.

### Beispiel 3: 4-Methyl-7-(1-propyl)inden (2)

45 ml einer 30-prozentigen Lösung von Natriummethanolat in Methanol (250 mmol) wurden bei 0 °C vorgelegt und innerhalb 30 min mit einer Mischung aus 14,2 g (100 mmol) 2,5-Octandion und 9 ml (110 mmol) Cyclopentadien versetzt. Nach 3 h Rühren bei Raumtemperatur wurde die Mischung in 300 ml Eiswasser gegossen, mit Diethylether extrahiert und die organische Phase über Magnesiumsulfat getrocknet. Der nach dem Eindampfen verbleibende Rückstand wurde an Kieselgel chromatographiert. Mit Hexan als Laufmittel ließen sich 6,8 g (39 %) des Indens 2 isolieren.
NMR-Daten siehe Tabelle 1.

### Beispiel 4:

Es wurde analog Beispiel 3 verfahren. Als Base wurden 250 mmol Kaliumtertiärbutylat in 50 ml Methanol verwendet. Es wurden 7,2 g (42 %) des Indens 2 isoliert.

### Beispiel 5:

Es wurde analog Beispiel 3 verfahren. Als Base wurden 250 mmol Kaliumhydroxid-Pulver in 50 ml Methanol verwendet. Es wurden 9,0 g (52 %) des Indens 2 isoliert.

### Beispiel 6: 4-Ethyl-7-(1-pentyl)inden (3)

Eine Lösung von 28,0 g (250 mmol) Kaliumtertiärbutylat in 50 ml Methanol wurde bei 0 °C innerhalb 20 min. mit einer Mischung aus 17,0 g (100 mmol) 2,5-Dekandion und 9,0 ml (110 mmol) Cyclopentadien versetzt. Nach 3 h Rühren bei 0°C und weiteren 2 h Rühren bei Raumtemperatur wurde die Lösung mit Wasser versetzt und mit Methylenchlorid extrahiert. Nach dem Trocknen über Magnesiumsulfat wurde die organische Phase eingedampft und der Rückstand an Kieselgel chromatographiert.
Mit Hexan als Laufmittel ließen sich 12,5 g (62 %) des Indens 3 isolieren. NMR-Daten siehe Tab. 1.

### Beispiel 7: 4-Methyl-7-(1-hexyl)-inden (4)

3,5 g (0,15 mol) Natrium wurden in 100 ml trockenem Methanol gelöst und auf 0 °C abgekühlt. Zu der Lösung tropfte man ein Gemisch von 12,8 g (0,06 mol) Undecandion und 5 ml (4,0 g, 0,06 mol) Cyclopentadien. Nachdem die Mischung noch 1 h bei 0 °C und 2 d bei Raumtemperatur gerührt worden war, hydrolysierte man mit 300 ml Wasser und extrahierte zweimal mit je 100 ml und zweimal mit je 50 ml Petrolether. Die vereinigten organischen Phasen wurden über MgSO₄ getrocknet, bis zur Trockene eingeengt und der Rückstand in ca. 30 ml Methanol aufgenommen.
Bei -20 °C fiel 4 in würfelförmigen Kristallen aus, die sich bei Raumtemperatur zu einem gelben Öl verflüssigten.
Ausb. 5,3 g (44 %). Es wurden zwei Isomere im Verhältnis 5:3 hergestellt;
IR (Film): ν = 2955-2857 (n-CH₂, CH₃), 1456, 1457, 1379, 1026, 1020, 813 (2 benachbarte aromatische C-H). MS (70 eV): m/z (%) = 129 (Methylinden, 80 ), 115 Indenyl, 77), 91 (Tropylium, 34), 77 (Phenyl, 33).

| | | | |
|---|---|---|---|
| C₁₆H₂₂(214,35) | Ber. | C 89,65 | H 10,35 |
| | Gef. | C 87,90 | H 10,17 |

NMR-Daten siehe Tab. 1.

### Beispiel 8: 4-Methyl-7-(1-octyl)inden (5)

Eine Lösung von 28,0 g (250 mmol) Kalium-t-butylat in 50 ml Methanol wurde bei 0 °C mit 9 ml (110 mmol) Cyclopentadien versetzt. Anschließend wurden bei 0 °C 21,2 g (100 mmol) 2,5-Tridekandion zugetropft. Nach 1 h Rühren bei 0 °C wurden noch weitere 3 h bei Raumtemperatur gerührt. Die Aufarbeitung erfolgte analog Beispiel 6. Die Säulenchromatographie lieferte 16,9 g (70 %) des Indens 5. NMR-Daten siehe Tab. 1.

### Beispiel 9: 4-Methyl-7-(2-propyl)-inden (6)

Analog Beispiel 6 wurden 14,2 g (100 mmol) 2-Methyl-3,6-heptandion umgesetzt. Die Säulenchromatographie lieferte 13,7 g (80 %) des Indens 6.
NMR-Daten siehe Tab. 1.

### Beispiel 10: 4-Methyl-7-(2-phenylethyl)inden (7)

Analog Beispiel 6 wurden 20,4 g (100 mmol) 1-Phenyl-3,6-heptandion umgesetzt. Die Säulenchromatographie lieferte 11,2 g (48 %) des Indens 7.
NMR-Daten siehe Tab. 1.

### Beispiel 11: 4-Methyl-7-phenylinden (8)

In einem Schlenkrohr wurden 5 g (0,21 mol) Natrium in 100 ml Methanol gelöst und auf 0 °C abgekühlt. Ein Gemisch aus 15,8 g (0,84 mol) 1-Phenyl-1,4-pentandion und 7,3 ml (5,8 g, 0,08 mol) Cyclopentadien wurde innerhalb von 15 min. zugetropft. Die rote Lösung wurde über Nacht bei Raumtemperatur gerührt, mit 200 ml Wasser hydrolysiert und fünfmal mit je 75 ml Petrolether extrahiert. Die vereinigten organischen Extrakte wurden filtriert, bis zur Trockene eingeengt und fraktioniert destilliert.
Ausb. 10,2 g (50 %) rotes Öl, Sdp. 135 °C.
NMR-Daten siehe Tab. 1.
IR (Film): ν = 3026 cm⁻¹, 2916, 1479, 818, 772, 763, 750, 698.

| | | | |
|---|---|---|---|
| C₁₆H₁₄ (206, 288) | Ber. | C 93,16 | H 6,84 |
| | Gef. | C 93,47 | H 7,07 |

### Beispiel 12: 4,7-Diphenylinden (9)

Eine Lösung von 10,0 g (42 mmol) 1,2-Dibenzoylethan in 10 ml Methanol wurde bei 0 °C mit 18,9 ml (105 mmol) einer 30-prozentigen Natriummethanolat-Lösung und 3,8 ml (46 mmol) Cyclopentadien versetzt. Nach 1,5 h Rühren bei 0 °C und 6 h Rühren bei Raumtemperatur wurde hydrolysiert und mit Methylenchlorid extrahiert. Die organische Phase wurde getrocknet und eingedampft. Der Rückstand wurde an Kieselgel chromatographiert. Mit einem Laufmittelgemisch aus Methylenchlorid/Hexan (1:1) ließen sich 3,81 g (34 %) des Indens 9 isolieren.
NMR-Daten siehe Tab. 1.

### Beispiel 13:

Es wurde wie in Beispiel 12 verfahren. Als Base wurde jedoch Kalium-t-butylat (105 mmol) in Methanol verwendet. Nach Säulenchromatographie erhielt man 32,9 g (35 %) des Indens 9.

### Beispiel 14: 4,7-Di-tert.-butylinden (10)

34,3 ml einer 30-prozentigen Lösung von Natriummethanolat (186 mmol) in Methanol wurden bei 0 °C innerhalb 10 min. mit einer Mischung aus 14,8 g (74,6 mmol) 2,2,7,7-Tetramethyl-3,6-octandion und 6,7 ml (82,1 mmol) Cyclopentadien versetzt. Nach 30 h Rühren bei Raumtemperatur wurde hydrolysiert und mit Methylenchlorid extrahiert. Nach dem Trocknen über MgSO₄ wurde bis zur Trockne eingedampft. Die Säulenchromatographie an Kieselgel mit Hexan als Laufmittel lieferte 1,75g
(10 %) des Indens 10.
NMR-Daten siehe Tab. 1.

### Beispiel 15:

Es wurde wie in Beispiel 14 verfahren. Es wurden jedoch 13,4 ml (164,2 mmol) Cyclopentadien eingesetzt. Nach Säulenchromatographie wurden 1,95 g (11,5 %) des Indens 10 erhalten.

### Beispiel 16: 4-Methyl-7-(p-chlorphenyl)inden (11)

Analog Beispiel 8 wurden 21,1 g (100 mmol) 1-(p-Chlorphenyl-1,4-pentandion umgesetzt. Die Säulenchromatographie mit Hexan/Methylenchlorid (10:1) als Laufmittel und anschließende Umkristallisation aus warmem Methanol lieferten 15,6g (65 %) des Indens 11. NMR-Daten siehe Tab. 1.

### Beispiel 17: 4-Methyl-7-(3-pyridyl)inden (12)

Analog Beispiel 6 wurden 17,7 g (100 mmol) 1-(3-Pyridyl)-1,4-pentandion umgesetzt. Die Säulenchromatographie mit Ethylacetat als Laufmittel lieferte 9,98 g (48 %) des Indens 12.
NMR-Daten siehe Tab. 1.

### Beispiel 18: 4-Methyl-7-(2-furyl)inden (13)

Analog Beispiel 8 wurden 16,6 g (100 mmol) 1-(2-Furyl)-1,4-pentandion umgesetzt. Die Säulenchromatographie mit Hexan/Methylenchlorid (7:1) als Laufmittel lieferte 13,7 g (70 %) des Indens 13.
NMR-Daten siehe Tab. 1.

### Beispiel 19: 4,7-Bis(2-furyl)inden (14)

18,9 ml einer 30-prozentigen Lösung von Natriummethanolat (105 mmol) in Methanol wurden bei 0 °C vorgelegt und innerhalb 1 h tropfenweise mit einer Lösung aus 9,2 g (42,2 mmol) 1,4-Bis-(2-furyl)-1,4-butandion und 3,8 ml (46,2 mmol) Cyclopentadien in DMSO versetzt. Nach 45 min. Rühren bei Raumtemperatur wurde die Mischung auf Eiswasser gegossen und mit Diethylether extrahiert. Nach dem Trocknen und Eindampfen wurde der Rückstand an Kieselgel chromatographiert. MIt Hexan/Methylenchlorid (2:1) als Laufmittel konnten 3,0g (29 %) des Indens 14 gewonnen werden.
NMR-Daten siehe Tab. 1.

### Beispiel 20: 4,7-Bis-(2-thiophen)inden (15)

14,7 ml einer 30-prozentigen Lösung von Natriummethanolat (81,7 mmol) in Methanol wurden bei 0 °C vorgelegt und innerhalb 1 h tropfenweise mit einer Lösung aus 8,15 g (32,6 mmol) 1,4-Bis(2-thiophen)-1,4-butandion und 2,9 ml (35,4 mmol) Cyclopentadien in DMSO versetzt. Nach 2h Rühren wurde die Mischung auf 300 ml Eiswasser gegossen, mit Diethylether extrahiert, getrocknet und eingedampft. Die Säulenchromatographie mit Hexan/Methylenchlorid (2:1) als Laufmittel lieferte 3,3 g (36 %) des Indens 15.
NMR-Daten siehe Tab. 1.

### Beispiel 21: 4-Ethylinden (16)

49,9 ml (250 mmol) einer 30-prozentigen Lösung von Natriummethanolat in Methanol wurden bei 0 °C vorgelegt und innerhalb 30 min. mit einer Mischung aus 13,2 g (100mmol) 4-Oxocapronaldehydhydrat und 9,0 ml (110 mmol) Cyclopentadien, gelöst in 5 ml Methanol, versetzt. Nach 2 h Rühren bei 0 °C und 2,5 h Rühren bei Raumtemperatur wurde hydrolyisert und mit Methylenchlorid extrahiert. Die organische Phase wurde über MgSO₄ getrocknet und eingeengt. Die Säulenchromatographie mit Hexan/Diisopropylether (100:1) lieferte 5,97 g (41 %) des Indens 16.
NMR-Daten siehe Tab. 1.

### Beispiel 22: 4-(1-Heptyl)inden (17)

20,4 ml einer 30-prozentigen Lösung von Natriummethanolat (111 mmol) in Methanol wurden bei 0 °C vorgelegt und innerhalb 30 min. mit einer Mischung aus 9,0 g (44,5 mmol) 4-Oxoundecylaldehyd und 4,0 ml (49 mmol) Cyclopentadien versetzt. Nach 2 h Rühren bei 0 °C und weiteren 3 h bei Raumtemperatur wurde hydrolysiert, mit Methylenchlorid versetzt und über Corolite filtriert. Die organische Phase wurde abgetrennt, über MgSO₄ getrocknet und eingedampft. Die Säulenchromatographie mit Hexan als Laufmittel lieferte 3,2 g (34 %) des Indens 17.
NMR-Daten siehe Tab. 1

### Beispiel 23: 1-(2,4,4-Trimethylpentyl)inden (18)

19,1 ml einer 30-prozentigen Lösung von Natriummethanolat (106 mmol) in Methanol wurden bei 0 °C vorgelegt und innerhalb 20 min. mit einer Mischung aus 8,4 g (42,4 mmol) 4-Oxo-6,8,8-trimethylnonylaldehyd und 3,8 ml (46,5 mmol) Cyclopentadien versetzt. Nach 4,5 h Rühren bei Raumtemperatur wurde die Mischung auf 150 ml Eiswasser gegossen und mit Diethylether extrahiert. Die organische Phase wurde getrocknet und eingedampft. Die Säulenchromatographie mit Hexan als Laufmittel lieferte 4,6 g (48 %) des Indens 18.
NMR-Daten siehe Tab. 1.

### Beispiel 24: 5,7-Diphenyl-4-(2-furyl)inden (19)

3,5 g (0,15 mol) Natrium wurden in 100 ml Methanol gelöst und auf 0 °C abgekühlt. Innerhalb von 30 min. wurde ein Gemisch aus 20,0 g (0,06 mol) 1-Furyl-2,4-phenyl-1,4-butandion und 5,0 ml (4,0 g, 0,06 mol) Cyclopentadien in 100 ml THF bei 0 °C zugetropft. Die Suspension wurde noch 1 h bei 0 °C und 2 d bei Raumtemperatur gerührt. Man hydrolysierte die dunkelrote Lösung mit 300 ml Wasser, wobei 19 schon als hellbrauner Feststoff ausfiel. Das Reaktionsgemisch wurde zweimal mit 100 ml und zweimal mit 50 ml Petrolether extrahiert, die organischen Phasen über MgSO₄ getrocknet, das Lösungsmittel abdestilliert und in 200 ml Methanol aufgenommen. Weiteres Produkt kristallisierte bei -20 °C aus. Ausb. 13,4 g (67 %), Schmp. 134 °C. MS (70 eV): m/z (%) = 334 (M+ ,39), 257 (M+-Phenyl, 37), 143 (Phenyl-furyl, 100), 113 (Indenyl, 69), 77 (Phenyl, 41). IR (KBr): ν 3050 cm⁻¹, 2963, 2924, 2889 (CH₂, Valenz), 1261, 1094, 1027, 1012, 803 (2 benachbarte aromatische C-H), 768, 733 (5 benachbarte aromatische C-H), 708, 702, 686.

| | | | |
|---|---|---|---|
| C₂₅H₁₈O (334,42) | Ber. | C 89,79 | H 5,43 |
| | Gef. | C 89,27 | H 5,48 |

### Beispiel 25: 2,4,7- bzw. 3,4,7-Trimethylinden (20) bzw. (21)

11,5 g (0,52 mol) Natrium wurden in 150 ml absolutem Methanol gelöst und anschließend auf 55 °C erhitzt. Ein Gemisch aus 18,3 g (0,25 mol) Methylcyclopentadien und 28,5g(0,25 mol) 2,5-Hexandion wurde langsam zugetropft. Nach beendeter Zugabe wurde noch zwei Stunden bei 55 °C gerührt und anschließend bei Raumtemperatur mit 100 ml Wasser hydrolysiert. Nach Zugabe von 150 ml Diethylether wurde die organische Phase im Scheidetrichter abgetrennt und die wäßrige Phase zweimal mit 50 ml Diethylether gewaschen. Die vereinigten organischen Phasen wurden über Natriumsulfat getrocknet, anschließend wurde das Lösungsmittel im Vakuum entfernt und der ölige Rückstand fraktionierend destilliert. Die Fraktion bei 58-67 °C (0,1 Torr) enthielt ein Isomerengemisch aus 2,4,7- bzw. 3,4,7-Trimethylinden (20,21) im Verhältnis 3:10. Ausbeute 7.3 g (19 %).
NMR-Daten siehe Tab. 1.

### Beispiel 26:

Zu einer Lösung von 8,6 g (375 mmol) Natrium in 200 ml Methanol wurde bei 0 °C innerhalb 1 h eine Mischung aus 12 g (150 mmol) Methylcyclopentadien und 17,1 g (150 mmol) 2,5-Hexandion zugetropft. Nach 18 h Rühren bei Raumtemperatur wurde die dunkelrote Mischung auf Eiswasser gegossen und ausgeethert. Nach dem Trockenen über Na₂SO₄ wurde das Lösemittel abgezogen und das verbleibenden Öl an 600 g Kieselgel (lange Säule) chromatographiert. Mit Hexan als Laufmittel konnten dicht aufeinander folgend zunächst 3,0 g (13 %) 3,4,7-Trimethylinden (21) und anschließend 1,5 g (6 %) 2,4,7-Trimethylinden (20) eluiert werden. Die anschließende Umkristallisation aus Hexan lieferte die reinen Produkte.
NMR-Daten siehe Tab. 1.

### Beispiel 27:

Zu einer Lösung von 6,4 g (280 mmol) Natrium in 100 ml Methanol wurden zunächst bei 0 °C 10,0 g (125 mmol) Methylcyclopentadien zugetropft. Diese Mischung wurde bei Raumtemperatur zu einer Lösung von 13,1 ml (112 mmol) 2,5-Hexandion in 50 ml Methanol innerhalb 1 h zugetropft. Nach 4 h Rühren bei Raumtemperatur wurde auf Eiswasser gegossen und bis pH 2 angesäuert. Danach wurde mit Diethylether extrahiert, über Na₂SO₄ getrocknet und eingedampft. Der Rückstand wurde an 600 g Kieselgel (lange Säule) mit Hexan/Methylenchlorid (20:1) chromatographiert. Man erhielt 0.90 g (5 %) 3,4,7-Trimethylinden (21) und 0,4 g (2 %) 2,4,7-Trimethylinden (20).
NMR-Daten siehe Tab. 1.

### Beispiel 28:

Eine Lösung von 32,0 g (280 mmol) Kalium-tert.-butylat in 300 ml Methanol wurde bei 0 °C mit einer Mischung aus 12,8 g (112 mmol) 2,5-Hexandion und 10,0 g (125 mmol) Methylcyclopentadien innerhalb 1 h versetzt. Nach 10 h Rühren bei Raumtemperatur wurde die Mischung auf HCl-saures Eiswasser gegossen und mehrmals mit Diethylether extrahiert. Der nach Trocknen und Einengen verbleibende Rückstand wurde an 200 g Kieselgel chromatographiert. Mit Hexan/Methylenchlorid (10:1) als Laufmittel wurden die beiden Isomere 20 und 21 gemeinsam eluiert. Ausbeute 4,0 g (23 %). Isomerenverhältnis 20:21=1:3.

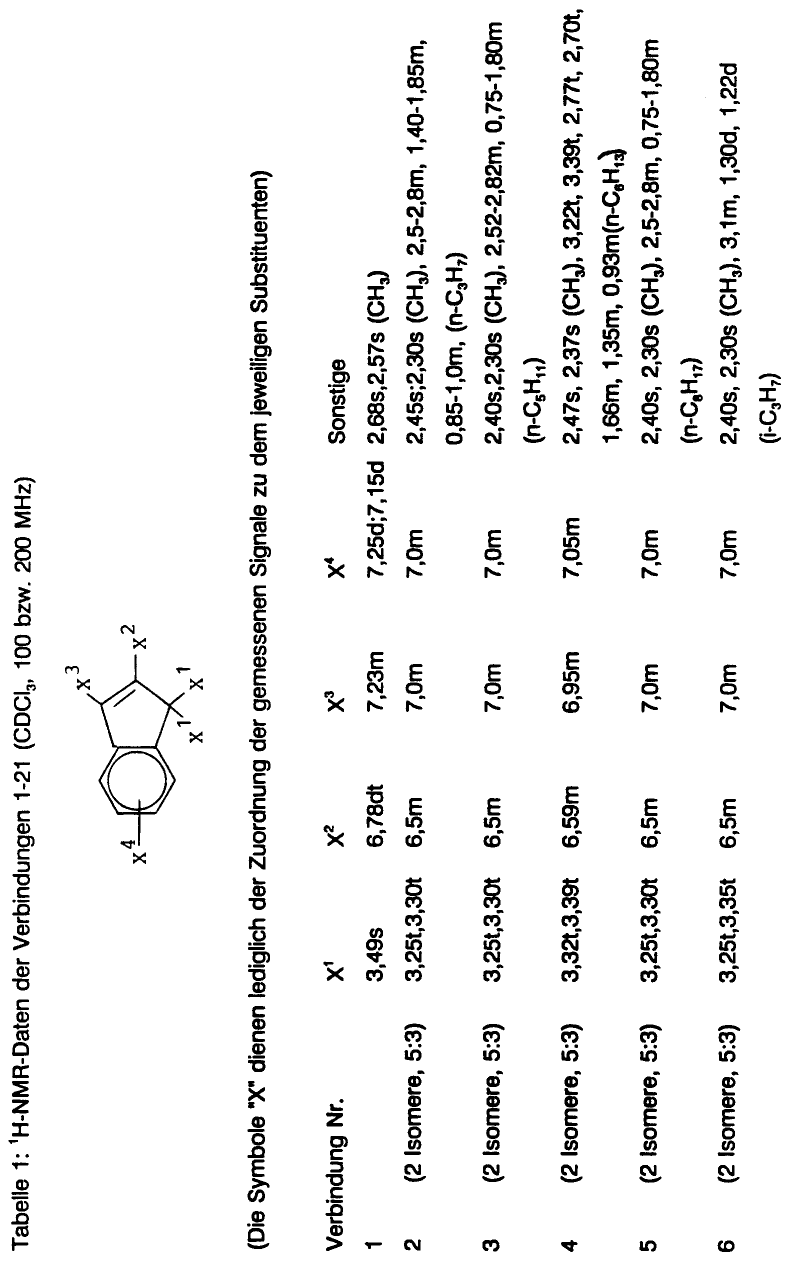

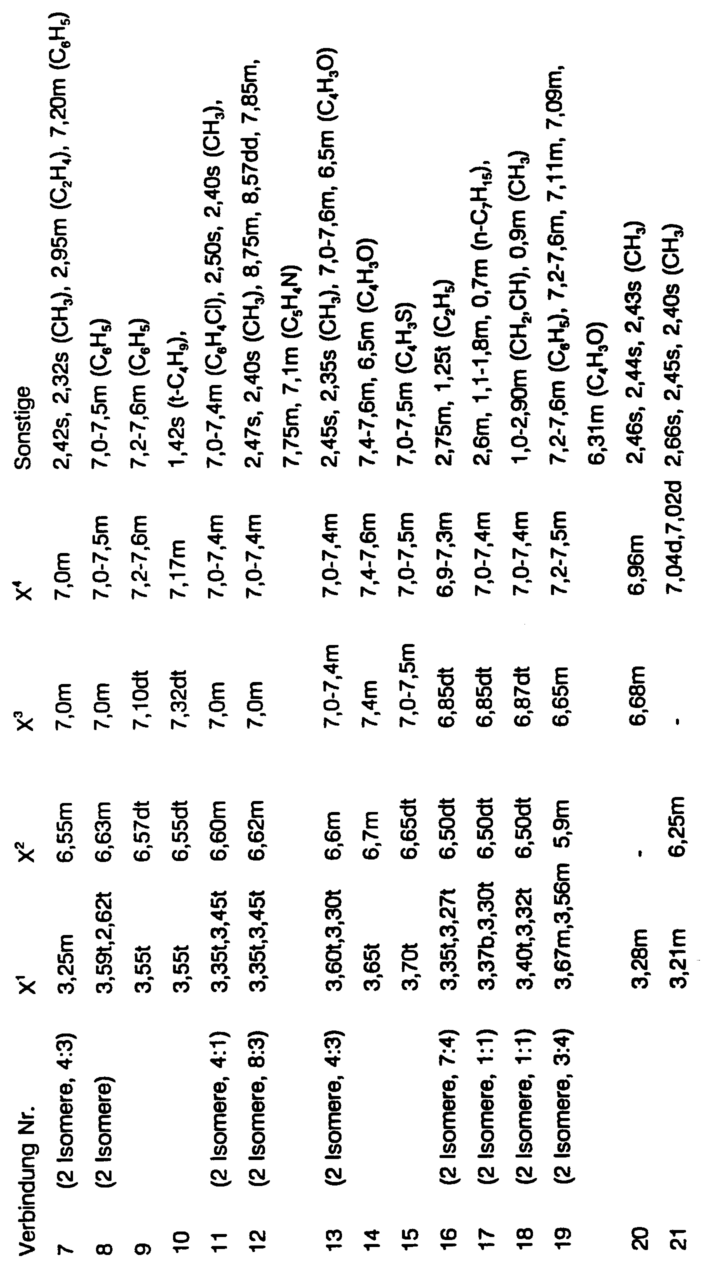

## Patentansprüche

1. Verfahren zur Herstellung einer Verbindung der Formel I oder deren Isomer der Formel Ia 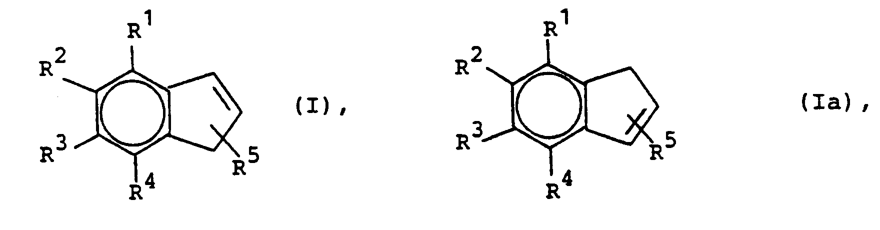 worin
R¹ (C₁-C₂₀)Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest -SiR⁶₃ oder einen heteroaromatischen Rest mit 5 oder 6 Ringgliedern, der ein oder mehrere Heteroatome enthalten kann, bedeutet,
R², R³ und R⁴ gleich oder verschieden sind und neben Wasserstoff die für R¹ genannten Bedeutungen besitzen,
R⁵ Wasserstoff, (C₁-C₁₀)Alkyl, (C₁-C₁₀)Fluoralkyl oder (C₂-C₁₀)Alkenyl bedeutet und
R⁶ für (C₁-C₁₀)Alkyl steht,
dadurch gekennzeichnet, daß man eine Verbindung der Formel II 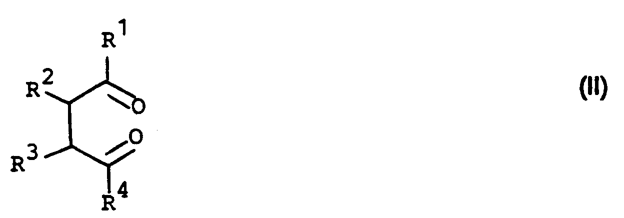 mit einer Verbindung der Formel III 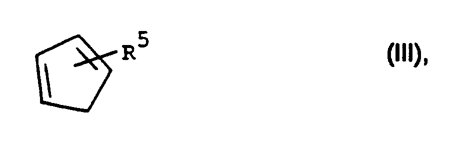 wobei die Substituenten R¹-R⁵ die genannten Bedeutungen besitzen, in Gegenwart einer Base umsetzt.

2. Verfahren gemäß Anspruch 1, dadurch gekennzeichnet, daß in der Formel I oder der Formel Ia
R¹ (C₁-C₁₀)Alkyl, Phenyl, (C₁-C₄)Alkoxy, (C₂-C₆)Alkenyl, (C₇-C₁₀)Arylalkyl, (C₇-C₁₀)Alkylaryl, Phenoxy, (C₁-C₆)Fluoralkyl, Halogenphenyl, (C₂-C₆)Alkinyl, einen Rest -SiR⁶₃ oder einen heteroaromatischen Rest mit 5 oder 6 Ringgliedern, der ein oder mehrere Sauerstoff-, Schwefel- und/oder Stickstoffatome enthalten kann, bedeutet,
R², R³ und R⁴ gleich oder verschieden sind und neben Wasserstoff die für R¹ genannten Bedeutungen besitzen,
R⁵ Wasserstoff, (C₁-C₆)Alkyl, (C₁-C₆)Fluoralkyl oder (C₂-C₆)Alkenyl bedeutet und
R⁶ für (C₁-C₆)Alkyl steht.

3. Verfahren gemäß Anspruch 1 oder 2, dadurch gekennzeichnet, daß in der Formel I oder der Formel Ia
R¹ (C₁-C₁₀)Alkyl, Phenyl, C₂-Alkenyl, (C₇-C₁₀)Arylalkyl, (C₇-C₁₀)Alkylaryl, (C₁-C₆)Fluoralkyl, Halogenphenyl, einen Rest -SiR⁶₃ oder einen heteroaromatischen Rest mit 5 oder 6 Ringgliedern, der ein Sauerstoff-, Schwefel- oder Stickstoffatom enthält, bedeutet,
R², R³ und R⁴ gleich oder verschieden sind und neben Wasserstoff die für R¹ genannten Bedeutungen besitzen,
R⁵ Wasserstoff, (C₁-C₃)Alkyl, (C₁-C₃)Fluoralkyl oder (C₂-C₃)Alkenyl bedeutet und
R⁶ für Methyl oder Ethyl steht.

4. Verfahren gemäß einem oder mehreren der Ansprüche 1-3, dadurch gekennzeichnet, daß als Base Natriummethanolat, Kaliumtertiärbutylat oder Kaliumhydroxid verwendet wird.

5. Verfahren gemäß einem oder mehreren der Ansprüche 1-4, dadurch gekennzeichnet, daß das Molverhältnis der Ausgangsverbindungen II : III: Base = 1 : 1-1,5 : 2-3 beträgt.

6. Verbindung der Formel I oder deren Isomer der Formel Ia 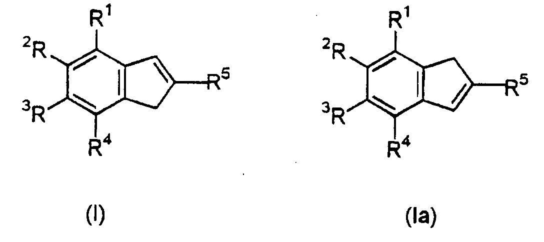 worin
R¹ C₁-C₂₀-Alkyl, C₆-C₁₄-Aryl, C₆-C₁₀-Halogenaryl oder einen heteroaromatischen Rest mit 5 oder 6 Ringgliedern, der ein oder mehrere Heteroatome enthalten kann, bedeutet,
R² und R⁴ Wasserstoff bedeuten
R³ Wasserstoff oder C₁-C₁₀-Alkyl bedeutet und
R⁵ C₁-C₁₀-Alkyl bedeutet.

7. Verbindung gemäß Anspruch 6, worin
R¹ C₆-C₁₄-Aryl, C₆-C₁₀-Halogenaryl oder einen heteroaromatischen Rest mit 5 oder 6 Ringgliedern, der ein oder mehrere Heteroatome enthalten kann, bedeutet, und
R³ Wasserstoff bedeutet.

8. Verbindung gemäß Anspruch 6 oder 7, worin R¹ C₆-C₁₄-Aryl bedeutet.

9. Verbindung gemäß Anspruch 6 oder 7, worin R¹ einen heteroaromatischen Rest mit 5 oder 6 Ringgliedern, der ein oder mehrere Heteroatome enthalten kann, bedeutet.

10. Verbindung gemäß Anspruch 6, worin
R¹ und R³ gleich oder verschieden sind und C₁-C₁₀-Alkyl bedeuten.

## Claims

1. A process for the preparation of a compound of the formula I or an isomer thereof of the formula Ia 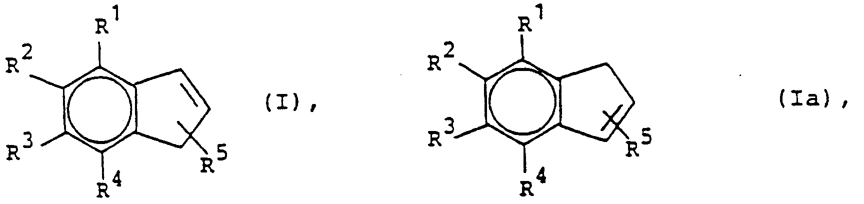 in which
R¹ is (C₁-C₂₀)alkyl, (C₆-C₁₄)aryl, (C₁-C₁₀)-alkoxy, (C₂-C₁₀)alkenyl, (C₇-C₂₀)arylalkyl, (C₇-C₂₀)alkylaryl, (C₆-C₁₀)aryloxy, (C₁-C₁₀)fluoroalkyl, (C₆-C₁₀)haloaryl, (C₂-C₁₀)alkynyl, a radical -SiR⁶₃ or a heteroaromatic radical having 5 or 6 ring members, which can contain one or more heteroatoms,
R², R³ and R⁴ are identical or different and, in addition to hydrogen, have the meanings given for R¹,
R⁵ is hydrogen, (C₁-C₁₀)alkyl, (C₁-C₁₀)-fluoroalkyl or (C₂-C₁₀)alkenyl and
R⁶ is (C₁-C₁₀)alkyl,
which comprises reacting a compound of the formula II 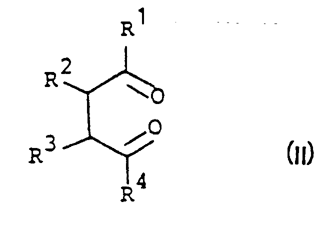 with a compound of the formula III 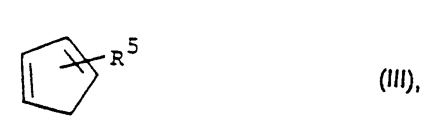 the substituents R¹-R⁵ having the meanings given, in the presence of a base.

2. The process as claimed in claim 1, wherein, in the formula I or the formula Ia,
R¹ is (C₁-C₁₀)alky, phenyl, (C₁-C₄)alkoxy, (C₂-C₆)alkenyl, (C₇-C₁₀)arylalkyl (C₇-C₁₀) alkylaryl, phenoxy, (C₁-C₆) fluoroalkyl, halophenyl, (C₂-C₆)alkynyl, a radical -SiR⁶₃ or a heteroaromatic radical having 5 or 6 ring members, which can contain one or more oxygen, sulfur and/or nitrogen atoms,
R², R³ and R⁴ are identical or different and, in addition to hydrogen, have the meanings given for R¹,
R⁵ is hydrogen, (C₁-C₆)alkyl, (C₁-C₆)fluoroalkyl or (C₂-C₆)alkenyl and
R⁶ is (C₁-C₆)alkyl.

3. The process as claimed in claim 1 or 2, wherein, in the formula I or the formula Ia,
R¹ is (C₁-C₁₀)alkyl, phenyl, C₂-alkenyl, (C₇-C₁₀)arylalkyl, (C₇-C₁₀)alkylaryl, (C₁-C₆) fluoroalkyl, halophenyl, a radical -SiR⁶₃ or a heteroaromatic radical having 5 or 6 ring members, which contains an oxygen, sulfur or nitrogen atom,
R², R³ and R⁴ are identical or different and, in addition to hydrogen, have the meanings given for R¹,
R⁵ is hydrogen, (C₁-C₃)alkyl, (C₁-C₃)fluoroalkyl or (C₂-C₃)alkenyl and
R⁶ is methyl or ethyl.

4. The process as claimed in one or more of claims 1-3, wherein sodium methanolate, potassium tert-butylate or potassium hydroxide is used as the base.

5. The process as claimed in one or more of claims 1-4, wherein the molar ratio of the starting compounds II:III: base is 1:1-1.5:2-3.

6. A compound of the formula I or an isomer thereof of the formula Ia 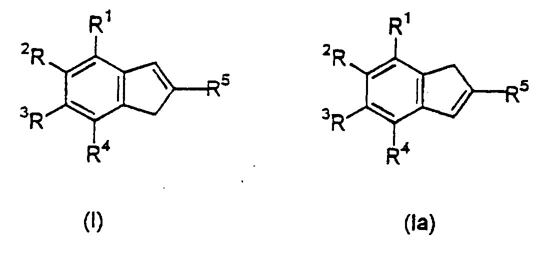 in which
R¹ is C₁-C₂₀-alkyl, C₆-C₁₄-aryl, C₆-C₁₀-haloaryl or a heteroaromatic radical having 5 or 6 ring members, which can contain one or more heteroatoms,
R² and R⁴ are hydrogen,
R³ is hydrogen or C₁-C₁₀-alkyl and
R⁵ is C₁-C₁₀-alkyl.

7. A compound as claimed in claim 6, in which
R¹ is C₆-C₁₄-aryl, C₆-C₁₀-haloaryl or a heteroaromatic radical having 5 or 6 ring members, which can contain one or more heteroatoms, and
R³ is hydrogen.

8. A compound as claimed in claim 6 or 7, in which R¹ is C₆-C₁₄-aryl.

9. A compound as claimed in claim 6 or 7, in which R¹ is a heteroaromatic radical having 5 or 6 ring members, which can contain one or more heteroatoms.

10. A compound as claimed in claim 6, in which R¹ and R³ are identical or different and are C₁-C₂₀-alkyl.

## Revendications

1. Procédé de préparation d'un composé de formule I ou de son isomère de formule Ia 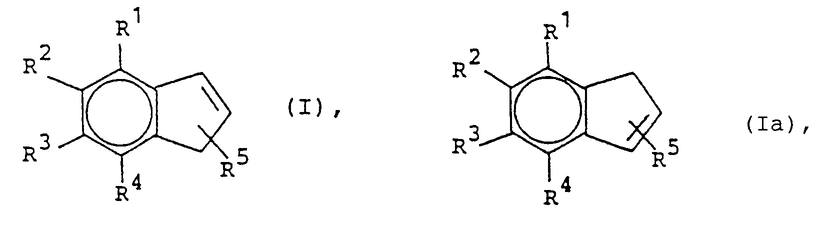 dans lesquelles
R¹ représente alkyle en C₁-C₂₀, aryle en C₆-C₁₄, alcoxy en C₁-C₁₀, alcényle en C₂-C₁₀, arylalkyle en C₇-C₂₀, alkylaryle en C₇-C₂₀, aryloxy en C₆-C₁₀, fluoroalkyle en C₁-C₁₀, halogénoaryle en C₆-C₁₀, alcynyle en C₂-C₁₀, un radical -SiR⁶₃ ou un radical hétéroaromatique à 5 ou 6 chaînons dans le cycle, qui peut contenir un ou plusieurs hétéroatomes, R², R³ et R⁴ sont identiques ou différents et, outre la signification hydrogène, possèdent les significations mentionnées pour R¹,
R⁵ représente l'hydrogène, alkyle en C₁-C₁₀, fluoroalkyle en C₁-C₁₀ ou alcényle en C₂-C₁₀ et
R⁶ représente alkyle en C₁-C₁₀,
caractérisé en ce qu'on fait réagir un composé de formule II 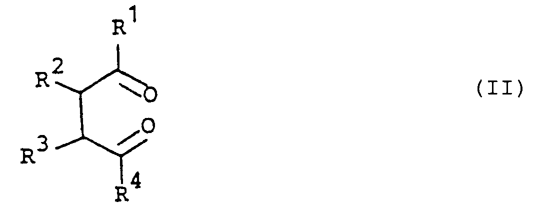 Avec un composé de formule III 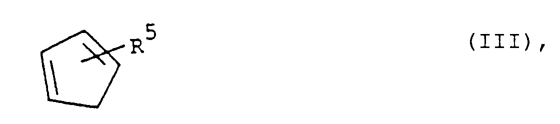 les substituants R¹ à R⁵ possédant les significations indiquées, en présence d'une base.

2. Procédé selon la revendication 1, caractérisé en ce que, dans la formule I ou dans la formule Ia,
R¹ représente alkyle en C₁-C₁₀, phényle, alcoxy en C₁-C₄, alcényle en C₂-C₆, arylalkyle en C₇-C₁₀, alkylaryle en C₇-C₁₀, phénoxy, fluoroalkyle en C₁-C₆, halogènophényle, alcynyle en C₂-C₆, un radical -SiR⁶₃ ou un radical hétéroaromatique avec 5 ou 6 chaînons dans le cycle, qui peut contenir un ou plusieurs atomes d'oxygène, de soufre et/ou d'azote,
R², R³ et R⁴ sont identiques ou différents et possèdent, outre la signification hydrogène, les significations mentionnées pour R¹,
R⁵ représente l'hydrogène, alkyle en C₁-C₆, fluoroalkyle en C₁-C₆ ou alcényle en C₂-C₆ et
R⁶ représente alkyle en C₁-C₆.

3. Procédé selon la revendication 1 ou 2, caractérisé en ce que, dans la formule I ou dans la formule Ia,
R¹ représente alkyle en C₁-C₁₀, phényle, alcényle en C₂, arylalkyle en C₇-C₁₀_{,} alkylaryle en C₇-C₁₀, fluoroalkyle en C₁-C₆, halogénophényle, un radical -SiR⁶₃ ou un radical hétéroaromatique avec 5 ou 6 chaînons dans le cycle, qui contient un atome d'oxygène, de soufre ou d'azote,
R², R³ et R⁴ sont identiques ou différents et possèdent, outre la signification hydrogène, les significations mentionnées pour R¹,
R⁵ représente l'hydrogène, alkyle en C₁-C₃, fluoroalkyle en C₁-C₃ ou alcényle en C₂-C₃ et
R⁶ représente méthyle ou éthyle.

4. Procédé selon l'une ou plusieurs des revendications 1 à 3, caractérisé en ce qu'on utilise comme base le méthanolate de sodium, le tert-butylate de potassium ou l'hydroxyde de potassium.

5. Procédé selon l'une ou plusieurs des revendications 1 à 4, caractérisé en ce que la proportion molaire des produits de départ II:III base est de 1:1 à 1,5:2 à 3.

6. Composé de formule I ou son isomère de formule Ia 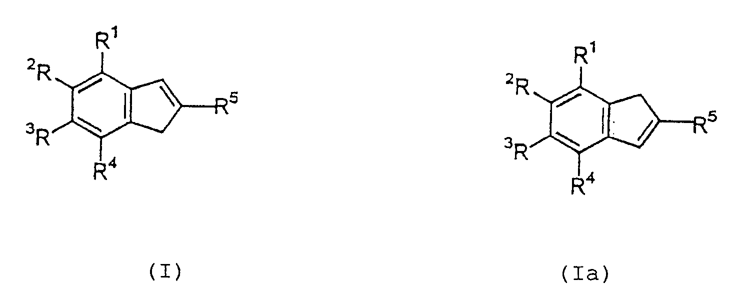 Dans lesquelles
R¹ représente alkyle en C₁-C₂₀, aryle en C₆-C₁₄, halogénoaryle en C₆-C₁₀ ou un radical hétéroaromatique avec 5 ou 6 chaînons dans le cycle, qui peut contenir un ou plusieurs hétéroatomes,
R² et R⁴ représentent l'hydrogène,
R³ représente l'hydrogène ou alkyle en C₁-C₁₀ et
R⁵ représente alkyle en C₁-C₁₀.

7. Composé selon la revendication 6, dans lequel
R¹ représente aryle en C₆-C₁₄, halogénoaryle en C₆-C₁₀ ou un radical hétéroaromatique avec 5 ou 6 chaînons dans le cycle, qui peut contenir un ou plusieurs hétéroatomes, et
R³ représente l'hydrogène.

8. Composé selon la revendication 6 ou 7, dans lequel R¹ représente aryle en C₆-C₁₄.

9. Composé selon la revendication 6 ou 7, dans lequel R¹ représente un radical hétéroaromatique avec 5 ou 6 chaînons dans le cycle, qui peut contenir un ou plusieurs hétéroatomes.

10. Composé selon la revendication 6, dans lequel R¹ et R³ sont identiques ou différents et représentent alkyle en C₁-C₂₀.
